Europäisches Patentamt

European Patent Office

Office européen des brevets

(19)

(11) Veröffentlichungsnummer : **0 086 539**
**B2**

(12) NEUE EUROPÄISCHE PATENTSCHRIFT

(45) Veröffentlichungstag der neuen Patentschrift :
02.11.89

(51) Int. Cl.⁴ : **C 12 M   1/12**

(21) Anmeldenummer : 83200211.7

(22) Anmeldetag : 10.02.83

(54) Vorrichtung zum Züchten von Microorganismen.

(30) Priorität : 12.02.82 CH 965/82

(43) Veröffentlichungstag der Anmeldung :
24.08.83 Patentblatt 83/34

(45) Bekanntmachung des Hinweises auf die Patenterteilung : 09.10.85 Patentblatt 85/41

(45) Bekanntmachung des Hinweises auf die Entscheidung
über den Einspruch : 02.11.89 Patentblatt 89/44

(84) Benannte Vertragsstaaten :
AT DE FR GB IT NL

(56) Entgegenhaltungen :
CH--A-- 602 161
DE--A-- 2 719 742
DE--A-- 2 816 925
FR--A--  430 290
FR--A--  820 743
FR--A-- 1 380 631
FR--E--  96 320
US--A-- 1 510 863
US--A-- 1 822 006
US--A-- 3 168 467
US--A-- 3 379 312
US--A-- 3 739 915
US--A-- 4 234 417
BIOTECHNOLOGY AND BIOENGINEERING, Band XX,
1978, Seiten 709-726, 1978, John Wiley & Sons, Inc.,
ARGYRIOS MARGARITIS et al.: "The rotorfermentor.
I. Description of the apparatus, power requirements,
and mass transfer characteristics"
Aiba et al., Biochemical Engineering, Academic
Press, Fig. 11.1, S. 303 und 304

(73) Patentinhaber : Chemap AG
Hölzliwisenstrasse 5
CH-8604 Volketswil (CH)

(72) Erfinder : Müller, Hans, Dr. Ing.
im Allmendli
CH-8703 Erlenbach (CH)

(74) Vertreter : Meyer, Ludgerus
Patentanwälte Meyer, Stach, Vonnemann Jungfernstieg 38
D-2000 Hamburg 36 (DE)

EP 0 086 539 B2

## Beschreibung

Die Erfindung betrifft eine Vorrichtung zur Züchtung von Mikroorganismen unter gleichzeitiger Abtrennung und Entfernung der entstehenden Stoffwechselprodukte, mit einem im Inneren eines mechanisch gerührten Fermenterbehälters angeordneten Membranfilter zum Zurückhalten der Mikroorganismen, im unteren Teil des Fermenterbehälters befindlichen Luftzuführvorrichtungen sowie einer oberseitigen Luftabführöffnung.

Aus der EP-A-7 133 ist eine Vorrichtung zur Züchtung von Mikroorganismen bekannt, in welcher die Abtrennung und Entfernung der entstehenden Stoffwechselprodukte mittels eines im Innern des Fermenters befindlichen Membranfilters erfolgt. Der in Form einer Filterkerze gestaltete Membranfilter ist im Innern eines Leitrohres befestigt.

Es ist auch ein Fermenter zur Züchtung von Mikroorganismen bekannt, in welchem die Metaboliten durch einen Membranfilter im Innern des Fermenters kontinuierlich abgetrennt werden. Dabei rotiert eine Membran in der Weise, daß eine Turbulenz in ihrer Umgebung stattfindet (Biotechnol. and Bioeng. (1978) 20, 709-726).

Trotz der gegenüber bekannten Vorrichtungen der Art verbesserten Standzeiten der Membranen, treten bei unterschiedlichen Medien Verstopfungen der Membranen und damit eine Abnahme des Filtratabflusses im Lauf des Verfahrens auf. Diese Leistungsabnahme hat offenbar die Kondensationspolarisation als Ursache, die durch die Strömung und Bewegung der Membranen allein nicht genügend unterdrückt werden kann.

Aus der CH-PS 602 161 ist schließlich ein Filtrationsapparat mit einer in einem Gehäuse angeordneten, zwischen einer rotationskörperförmigen Außenfiltrationsfläche und einer in diese geschachtelten rotationskörperförmigen Innenfiltrationsfläche gebildeten, ringförmigen Filtrationskammer bekannt, bei welcher in der einen Suspensionszulauf und einen Produktauslaß aufweisenden Filtrationskammer eine rotierende Misch-Rühr-Vorrichtung angeordnet ist, die als rotationskörperförmiges Stabgitter oder als mit Rippen und Löchern versehene Rohrwand ausgebildet sein kann. Diese Vorrichtung wird jedoch mit ständigem Suspensionsdurchlauf betrieben und eignet sich auch nicht zur Züchtung von Mikroorganismen.

Aufgabe der Erfindung ist es nun, eine Vorrichtung zur Züchtung von Mikroorganismen der im Oberbegriff des Patentanspruchs 1 definierten Art zu schaffen, die mit einfachen Mitteln auch bei hoher Konzentration an Mikroorganismen ohne Schädigung derselben eine verlängerte Konstanthaltung der Filtrationsrate und eine verbesserte Standzeit ermöglicht.

Zur Lösung dieser Aufgabe ist die Vorrichtung der eingangs genannten Art mit den Merkmalen des Patentanspruchs 1 ausgestattet.

Durch Rühren in unmittelbarer Umgebung der Membran kann sich eine höhere Konzentration vor allem von Stoffen aus dem komplexen Fermentationsmedium kaum aufbauen. Die erforderliche Druckdifferenz zwischen der Konzentrat- und der Filtratseite kann durch Druck auf der Konzentratseite aufrechterhalten werden. Die Fermentierung unter leichtem Überdruck hat sich in vielen Fällen als vorteilhaft erwiesen.

Als Membranfilter selbst hat sich eine mit einer semipermeablen Membran überzogenen Sinterkerze als geeignet erwiesen. Diese ist in der Regel auf der Aussen- und Innenseite mit der Membran überzogen. Die Kerze kann vollständig gepackt oder hohl sein. Die Öffnungen der Poren können einen Durchmesser zwischen 0,1 und 50 Mikron aufweisen.

Die Rührvorrichtung ist aus Schikanen gebildet, die als eine Art Schikanenkorb den Membranfilter umgeben. Der Schikanenkorb besteht aus ebenen oder spiralförmigen, senkrecht oder im spitzen Winkel zur Antriebswelle auf Ringscheiben angeordneten Schikanen. Die Schikanen können zusätzlich mit nach aussen weisenden Rührarmen versehen sein.

Als Variante kann auch der Membranfilter selbst rotieren, wobei dann der Schikanenkorb feststeht.

Die Erfindung soll anhand von Zeichnungen näher beschrieben werden. Es zeigen :

Figur 1 einen Längsschnitt durch den Fermenter mit einem Membranfilter und erfindungsgemässer Rührvorrichtung,

Figur 2 die Rührvorrichtung mit nach aussen befestigten Rührarmen,

Figur 3 eine Variante eines Rührarmes.

Figur 4 einen Längsschnitt durch den Fermenter mit einem sich drehenden Membranfilter,

Figur 5 ein Membranfilter mit innen liegendem drehbaren Schikanenkorb,

Figur 6 ein Membranfilter, der von einem Ankerrührer mit Schikanen als Rührarme umgeben ist.

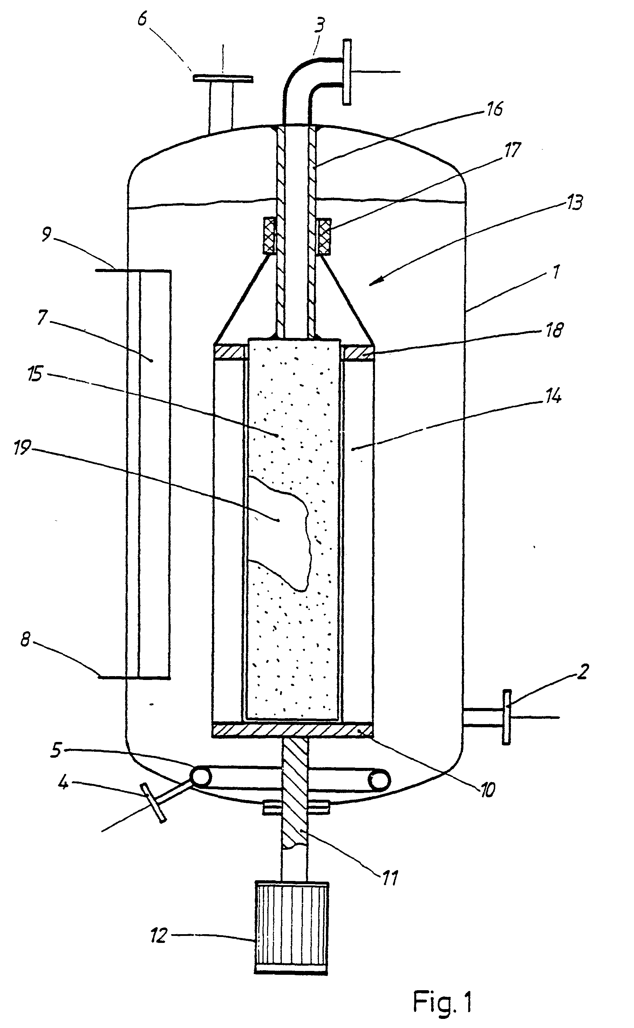

Der Fermenter gemäss Fig. 1 besteht aus einem Behälter 1 mit einem Stutzen 2 für den Substratzufluss und einem Stutzen 3 für den Filtratabfluss. Im unteren Teil des Fermenters befindet sich ein Anschlussstutzen 4 für die Luftzufuhr, ein Ringrohr 5 zur Luftverteilung, und im Deckel des Behälters 1 ein Stutzen 6 für die Abluft. Im Innern des Behälters 1 sind Kühlelemente 7 befestigt, die mit Stutzen für die Zufuhr 8 und die Abfuhr 9 des Kühlmittels vorgesehen sind. Eine Ringscheibe 10 wird über eine Welle 11 mittels eines Elektromotors 12 angetrieben. Mit der Ringscheibe 10 ist eine Rührvorrichtung 13 verbunden, die aus Schikanen 14 besteht und deren obere Befestigung aus einer weiteren Ringscheibe 18 gebildet wird. Die Schikanen 14 können eben oder aber auch spiralförmig oder als schraubenförmige Flügel gestaltet sein. Im Innern der Rührvorrichtung 13 ist ein rohrförmiger Membranfilter 15, der in seinem oberen Teil an einem Rohr 16 für den Ablauf des Filtrates befestigt ist. Die Führung des Filters im oberen Teil erfolgt über eine Lagerung 17. Der Membranfilter 15 kann aus einer porösen Filterkerze als Träger für eine semipermeable Membran 19 bestehen. Die Abdichtung von Konzentrat und Permeat erfolgt in bekannter Weise.

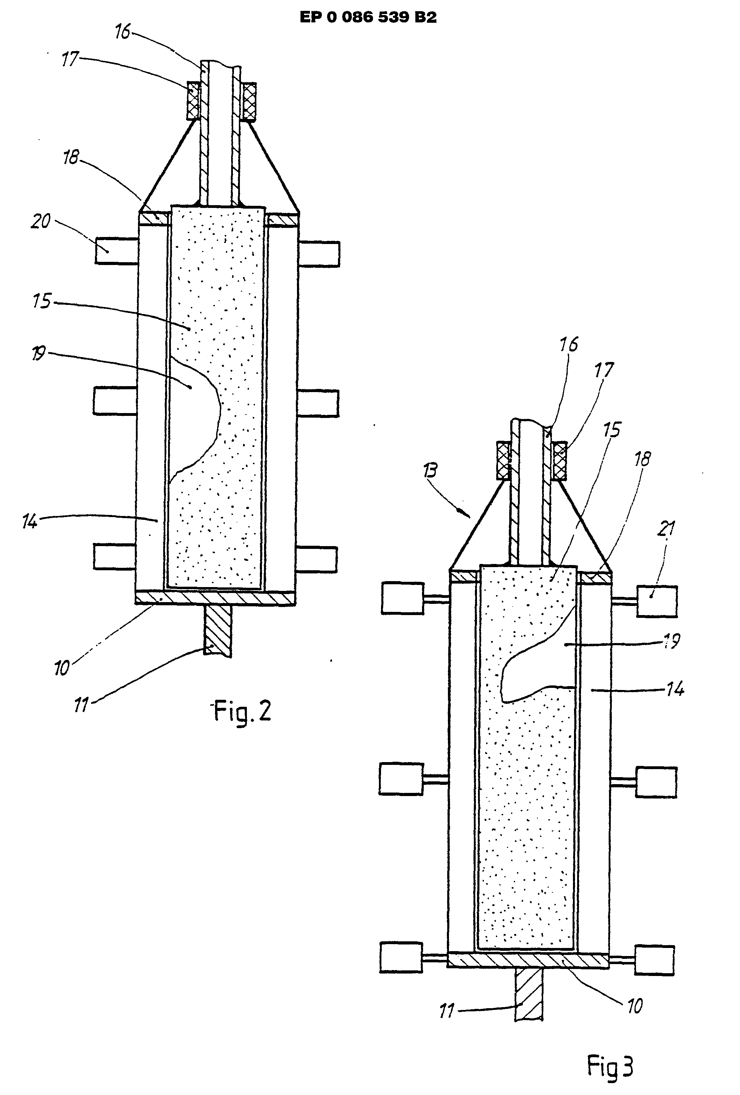

Fig. 2 zeigt die Rührvorrichtung 13 mit an der Aussenseite befestigten Rührflügeln 20. Diese können allein oder zusätzlich zu den inneren Schikanen vorgesehen sein.

Fig. 3 zeigt ein Rührblatt 21, welches als Turbinenrührer ausgebildet ist. Es kann senkrecht, aber auch in einem Winkel zur Drehachse verstellt sein.

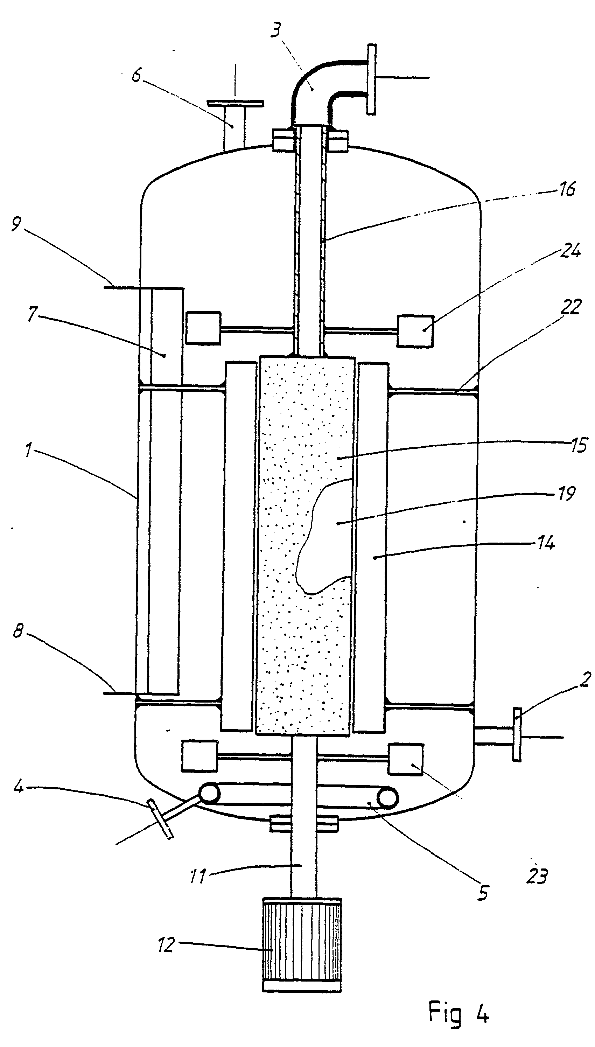

In Fig. 4 ist der Membranfilter 15 drehbar angeordnet, während die Schikanen 14 über Befestigungen 22 mit der Behälterwand 1 fest verbunden sind. In diesem Fall ist die obere Lagerung 17 des Membranfilters 15 im Deckel des Fermenters angebracht. Es können ein Fermenterrührer 23 unterhalb des Membranfilters 15 an der Antriebswelle 11 oder ein Rührer 24 darüber am drehbaren Abflussrohr 16 des Filtrates angebracht sein.

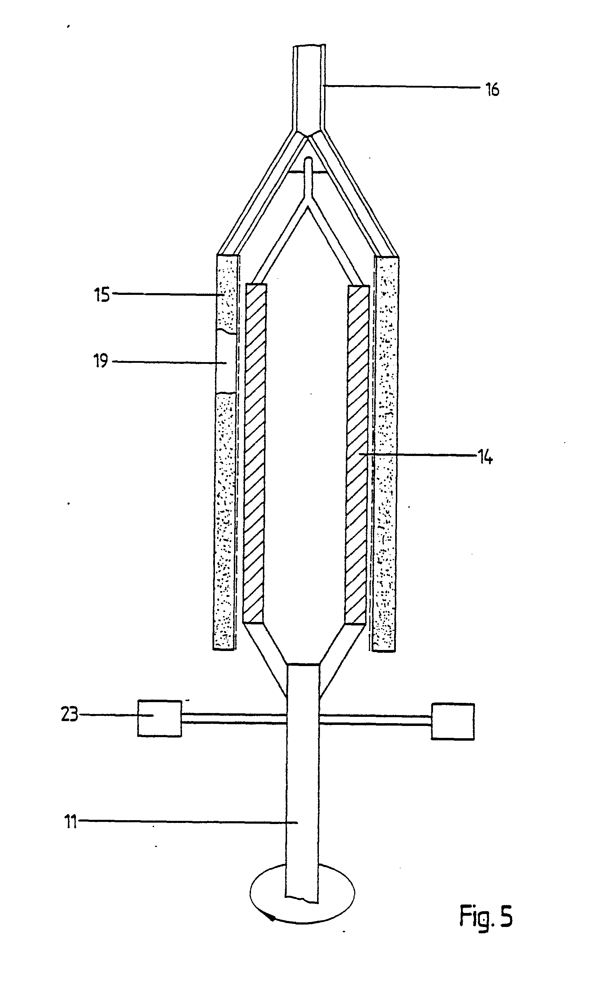

In Fig. 5 ist der Membranfilter 15 an dem Rohr 16 befestigt. Die semipermeable Membran 19 befindet sich im Innern des Sinterrohres. Die Aussenseite ist abgedichtet. Das Ultrafiltrat fliesst aus den Hohlräumen der Sinterkerze über ein Sammelrohr 16' in das Rohr 16.

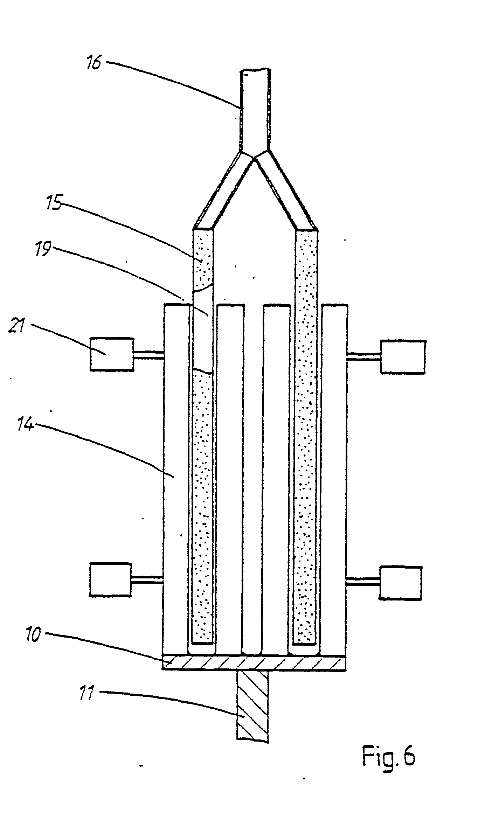

In Fig. 6 ist ein beidseitig mit Membranen 19 versehener Membranfilter 15 von einer als Schikanenkorb ausgebildeten Rührvorrichtung umgeben. An den Schikanen 14 können zusätzliche Rührarme 21 befestigt sein.

Im Betrieb der Fermenteranlage wird die Ringscheibe 10 über die Welle 11 und den Elektromotor 12 angetrieben. Die Stoffwechselprodukte der Mikroorganismen, dei sich in einem Nährsubstrat im Fermenter befinden, verlassen den Fermenter zusammen mit der Flüssigkeit über die Membranen 19, die Hohlräume des porösen Stützkörpers des Membranfilters 15 und das Abflussrohr 16. Die Mikroorganismen verbleiben im Fermenterbehälter 1. Durch die Rotation der Rührvorrichtung 13 mit den Schikanen 14 entsteht in unmittelbarer Nähe der Membran 19 eine so starke Turbulenz, dass die Membran 19 frei von Ablagerungen trotz hoher Konzentration an Mikroorganismen im Fermenter bleibt.

## Patentansprüche

1. Vorrichtung· zur Züchtung von Mikroorganismen unter gleichzeitiger Abtrennung und Entfernung der entstehenden Stoffwechselprodukte, mit einem im Inneren eines mechanisch gerührten Fermenterbehälters (1) angeordneten Membranfilter (15) zum Zurückhalten der Mikroorganismen, im unteren Teil des Fermenterbehälters (1) befindlichen Luftzuführvorrichtungen (4, 5) sowie einer oberseitigen Luftabführöffnung (2), dadurch gekennzeichnet, daß
a) der Membranfilter (15) als von einer semipermeablen Membran (19) überzogenes Sinterrohr mit Öffnungen zwischen 0,1 und 50 μm ausgebildet ist,
b) der Membranfilter (15) von einer Rührvorrichtung (13) umgeben ist und
c) die Rührvorrichtung (13) aus relativ zum Membranfilter (15) in unmittelbarer Nähe der Membran (19) turbulenzerzeugend rotierbaren, ebenen oder spiralförmig ausgebildeten Schikanen (14) besteht.

2. Vorrichtung nach Anspruch 1, bei welcher der Membranfilter (15) rotierbar gelagert ist, dadurch gekennzeichnet, daß der Membranfilter (15) von über Befestigungsmittel (22) an der Wand des Behälters (1) befestigten Schikanen (14) umgeben ist.

3. Vorrichtung nach Anspruch 1 oder 2, dadurch gekennzeichnet, daß die Schikanen (14) senkrecht oder unter einem spitzen Winkel zur Antriebswelle (11) angeordnet sind.

4. Vorrichtung nach einem der Ansprüche 1 bis 3, dadurch gekennzeichnet, daß die Schikanen (14) der Rührvorrichtung (13) an ihrer Außenseite mit Rührarmen (20) versehen sind.

5. Vorrichtung nach einem der Ansprüche 2 bis 4, dadurch gekennzeichnet, daß an der Antriebswelle (11) oberhalb und/oder unterhalb des Membranfilters (15) Rührelemente (23, 24) befestigt sind.

6. Vorrichtung nach Anspruch 1, dadurch gekennzeichnet, daß ein beidseitig mit Membranen (19) überzogener Membranfilter (15) von Schikanen (14) eines Ankerrührers umgeben ist.

7. Vorrichtung nach Anspruch 1, dadurch gekennzeichnet, daß die Schikanen (14) der Rührvorrichtung (13) an ihrer Unterseite an einem Ring (10) und an ihrer Oberseite an einem Ring (18) befestigt sind.

## Claims

1. A device for culturing micro-organisms with simultaneous separation and removal of the metabolic products present, having a membrane filter (15) arranged inside a mechanically agitated fermentation container (1) for retaining the micro-organisms, air-supply devices (4, 5) arranged in the lower part of the fermentation container (1) and an air-removal opening, (2) at the top, characterized in that
a) the membrane filter (15) is constructed in the form of a sintered tube covered with a semipermeable membrane (19) and having openings of between 0.1 and 50 μm,
b) the membrane filter (15) is surrounded by an agitation device (13), and
c) the agitation device (13) comprises flat or spiral-shaped baffle plates (14) rotatable relative to the membrane filter (15) in the immediate vicinity of the membrane (19) so as to produce turbulence.

2. A device according to Claim 1, in which the membrane filter (15) is mounted so as to be rotatable, characterized in that the membrane filter (15) is surrounded by baffle plates (14) secured to the wall of the container (1) by way of fastening means (22).

3. A device according to Claim 1 or 2, characterized in that the baffle plates (14) are arranged perpendicular or at an acute angle to the driving shaft (11).

4. A device according to any one of Claims 1 to 3, characterized in that the baffle plates (14) of the agitation device (13) are provided with agitation arms (20) on their exterior.

5. A device according to any one of Claims 2 to 4, characterized in that agitation elements (23, 24) are secured to the driving shaft (11) above and/or below the membrane filter (15).

6. A device according to Claim 1, characterized in that a membrane filter (15) covered on both sides with membranes (19) is surrounded by baffle plates (14) of an anchor agitator.

7. A device according to Claim 1, characterized in that the baffle plates (14) of the agitation device (13) are secured to a ring (10) on their underside and to a ring (18) on their upper side.

**Revendications**

1. Dispositif pour la culture de micro-organismes, sous séparation et élimination simultanées des produits métaboliques formés, comportant un filtre à membrane (15) qui est disposé à l'intérieur d'un réservoir fermenteur (1) à agitation mécanique, pour retenir les micro-organismes, ainsi que des dispositifs d'amenée d'air (4, 5) qui se trouvent dans la partie inférieure du réservoir fermenteur (1) et un orifice d'évacuation d'air situé du côté supérieur, caractérisé en ce que :

a) le filtre à membrane (15) est constitué d'un tube fritté revêtu d'une membrane semi-perméable (19) et pourvu d'ouverture d'un diamètre compris entre 0,1 et 50 μm.

b) le filtre à membrane (15) est entouré d'un dispositif agitateur (13), et

c) le dispositif agitateur (13) se compose de chicanes (14) rotatives, planes ou en forme de spirale, produisant une turbulence à proximité immédiate de la membrane (19) par rapport au filtre à membrane (15).

2. Dispositif selon la revendication 1, dans lequel le filtre à membrane (15) est monté rotatif, caractérisé en ce que le filtre à membrane (15) est entouré de chicanes (14) qui sont fixées, par l'intermédiaire d'organes de fixation (22), à la paroi du réservoir (1).

3. Dispositif selon la revendication 1 ou 2, caractérisé en ce que les chicanes (14) sont disposées verticalement ou en formant un angle aigu par rapport à l'arbre d'entraînement (11).

4. Dispositif selon l'une des revendications 1 à 3, caractérisé en ce que les chicanes (14) du dispositif agitateur (13) sont pourvues, sur leur côté extérieur, de bras agitateurs (20).

5. Dispositifs selon l'une des revendications 2 à 4, caractérisé en ce que sur l'arbre d'entraînement (11) sont fixés, au-dessus et/ou au-dessous du filtre à membrane (15), des éléments d'agitation (23, 24).

6. Dispositif selon la revendication 1, caractérisé en ce qu'un filtre à membrane (15), revêtu de membranes (19) sur ses deux faces, est entouré de chicanes appartenant à un agitateur à armature.

7. Dispositif selon la revendication 1, caractérisé en ce que les chicanes (14) du dispositif agitateur (13) sont fixées, par leur côté inférieur, à un anneau (10) et, par leur côté supérieur, à un anneau (18).

EP 0 086 539 B2

Fig. 1

Fig. 2

Fig 3

Fig 4

Fig. 5

4

Fig. 6